(19) Europäisches Patentamt
European Patent Office
Office européen des brevets

(11) Veröffentlichungsnummer: **0 014 844**
B1

(12) **EUROPÄISCHE PATENTSCHRIFT**

(45) Veröffentlichungstag der Patentschrift: **05.05.82**

(21) Anmeldenummer: **80100268.4**

(22) Anmeldetag: **21.01.80**

(51) Int. Cl.³: **C 09 B 62/08,** C 07 D 251/50 // C07D251/44

(54) **Fluortriazin-Derivate, Verfahren zur Herstellung von Fluortriazin-Derivaten und von Reaktivfarbstoffen und deren Verwendung.**

(30) Priorität: **31.01.79 DE 2903594**

(43) Veröffentlichungstag der Anmeldung: **03.09.80 Patentblatt 80/18**

(45) Bekanntmachung des Hinweises auf die Patenterteilung: **05.05.82 Patentblatt 82/18**

(84) Benannte Vertragsstaaten: **CH DE FR GB IT**

(56) Entgegenhaltungen:
**DE-A-2 711 150**
**DE-A-2 731 617**

(73) Patentinhaber: **BAYER AG, Zentralbereich Patente, Marken und Lizenzen, D-5090 Leverkusen 1, Bayerwerk (DE)**

(72) Erfinder: **Wunderlich, Klaus, Dr., Carl-Rumpff-Strasse 21, D-5090 Leverkusen 1 (DE)**
Erfinder: **Harms, Wolfgang, Dr., Walter-Flex-Strasse 21, D-5090 Leverkusen 1 (DE)**

**EP 0 014 844 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Fluortriazin-Derivate, Verfahren zur Herstellung von Fluortriazin-Derivaten und von Reatktivfarbstoffen und deren Verwendung

Gegenstand der Erfindung ist ein Verfahren zur Herstellung von Fluortriazin-Derivaten der Formel

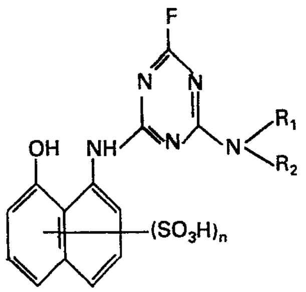

(I)

worin $R_1$ = H, gegebenenfalls substituiertes Alkyl, Cycloalkyl oder Aralkyl,

$R_2$ = H, gegebenenfalls substituiertes Alkyl,

n = 1 oder 2,

wobei die Alkylreste $R_1$ und $R_2$ auch zu einem heterocyclischen Ringsystem verknüpft sein können.

Bevorzugt ist die Herstellung von Verbindungen der Formel

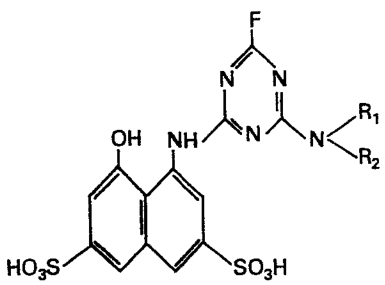

(II)

worin $R_1$ und $R_2$ die oben angegebene Bedeutung haben.

Weiterhin bevorzugt ist die Herstellung von Verbindungen der Formel

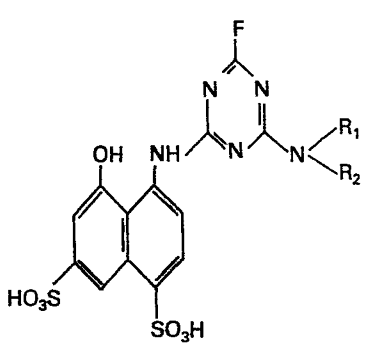

(3)

worin $R_1$ und $R_2$ die oben angegebene Bedeutung haben.

Das neuartige Herstellungsverfahren ist dadurch gekennzeichnet, dass man Aminohydroxynaphthalinsulfonsäuren der Formel

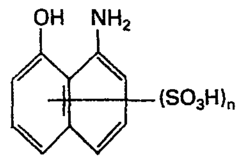

(4)

worin n = 1 oder 2 ist,

in wässriger Lösung oder Suspension in bekannter Weise vorzugsweise bei pH 3,5–4,5 mit 2,4,6-Trifluor-s-triazin (Cyanurfluorid) umsetzt und die erhaltene Lösung oder Suspension der 4,6-Difluor-s-triazinyl-amino-Verbindung der Formel

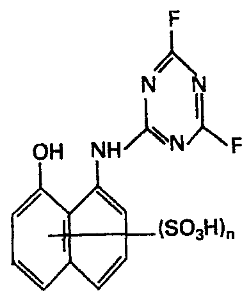

(5)

worin n = 1 oder 2 ist,

zu einer wässrigen Lösung oder Suspension eines Amines der Formel

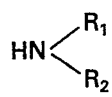

(6)

worin $R_1$ und $R_2$ die oben angegebene Bedeutung haben,

gibt und hierbei einen pH-Bereich zwischen 8,5 und 12,5 einhält oder direkt eine Verbindung der Formel (5) zu einer wässrigen Lösung oder Suspension des Amines (6) gibt und hierbei einen pH-Bereich zwischen 8,5 und 12,5 einhält.

Die Verbindungen der Formel (6) werden dabei im Molverhältnis 1:1 bis 4:1 oder im Fall, dass $R_1$ und $R_2$ Wasserstoff sind, bis 10:1 eingesetzt. Auch grössere Überschüsse schaden im allgemeinen nicht. Die optimalen Molverhältnisse und pH-Bereiche richten sich nach der Art des verwendeten Amins.

Im Reaktionsmedium können gegebenenfalls auch geringe Mengen solcher wassermischbarer Lösungsmittel vorhanden sein, die nicht mit dem Fluortriazin reagieren. Gegenstand der Erfindung ist weiterhin ein Verfahren zur Herstellung von Reaktivfarbstoffen der Formel

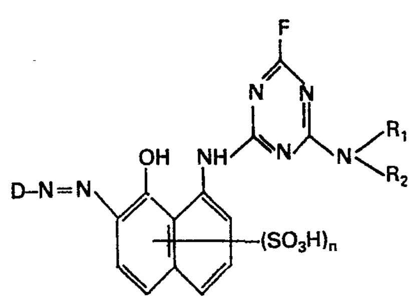

(7)

worin

$R_1$, $R_2$ und n die oben angegebene Bedeutung haben und wobei D für den Rest einer Diazokomponente steht. Dieses Verfahren ist dadurch gekennzeichnet, dass man die gemäss vorgenannten Verfahren herstellte Kupplungskomponente der Formel (1) mit diazotierten Aminen der Formel $D{-}NH_2$ (10) kuppelt, wobei D die oben angegebene Bedeutung besitzt.

Die nach dem erfindungsgemässen Verfahren erhaltenen Verbindungen der Formel (1) sind wertvolle Zwischenverbindungen für die Herstellung von Azo-Reaktivfarbstoffen. Besonders wertvoll sind die Verbindungen, die ausgehend von H-Säure und K-Säure erhalten werden. Durch die nachfolgende Kupplung mit einem diazotierten Arylamin gelangt man zu brillanten roten Monofluor-s-traizin-Farbstoffen der Formel (7), wie sie beispielsweise in der DE-AS 16 44 208 oder DE-OS 27 11 150 beschrieben werden.

Aus der DE-OS 27 47 011 und der DE-OS 27 29 240 ist ein Verfahren zur Herstellung von Verbindungen der Formel (2) bekannt geworden, in der 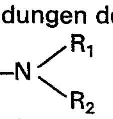 den Rest eines gegebenenfalls substituierten Arylamins bedeutet. Die Difluortriazinylamino-Verbindung der Formel (5) wird hierin nach ihrer in wässrigem Medium bei z.B. pH 3,5–4,5 erfolgten Herstellung durch Zugabe des Arylamins und Einstellung eines pH-Wertes von 5,5-6 zu den Verbindungen der Formel (2) umgesetzt, in der 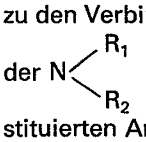 den Rest eines gegebenenfalls substituierten Arylamins bedeutet.

Verwendet man anstelle von Arylaminen Ammoniak, Alkyl- oder Aralkylamin erhält man unter diesen Bedingungen unbefriedigende Ergebnisse, auch wenn man den pH-Wert auf Werte oberhalb 8,5 erhöht.

Nach dem erfindungsgemässen Verfahren erhält man hingegen die gewünschten Verbindungen der Formel (1) in hoher Ausbeute und Reinheit. Nach anschliessender Kupplung, die vorteilhaft direkt im Reaktionsmedium durch Zugabe einer Diazonium-Verbindung in wässriger Lösung oder Suspension durchgeführt wird, können die reaktiven Azofarbstoffe der Formel (7) in hoher Ausbeute und Reinheit erhalten werden.

Als geeignete Aminohydroxynaphthalinsulfonsäure der Formel (4) seien beispielsweise genannt:

1-Amino-8-hydroxynaphthalin-3,6-disulfonsäure
1-Amino-8-hydroxynaphthalin-4,6-disulfonsäure
1-Amino-8-hydroxynaphthalin-2,4-disulfonsäure
1-Amino-8-hydroxynaphthalin-3,5-disulfonsäure
1-Amino-8-hydroxynaphthalin-3-sulfonsäure
1-Amino-8-hydroxynaphthalin-4-sulfonsäure
1-Amino-8-hydroxynaphthalin-6-sulfonsäure.

Beispiele für Amine der Formel (6) sind folgende:

Ammoniak, Methylamin, Äthylamin, n-Propylamin, Isopropylamin, n-Butylamin, Isobutylamin, sec. Butylamin, tert. Butylamin, Dimethylamin, Diäthylamin, Methyläthylamin, Allylamin, 2-Chloräthylamin, 2-Methoxyäthylamin, 2-Aminoäthanol, 2-Methylaminoäthanol, Bis-(2-hydroxyäthyl)-amin, 2-Acetylaminoäthylamin, 1-Amino-2-propanol, 3-Methoxypropylamin, 1-Amino-3-dimethylaminopropan, 3-Methylaminopropionsäurenitril, 2-Aminoäthansulfonsäure, 2-Methylaminoäthansulfonsäure, 2-Sulfatoäthylamin, Aminoessigsäure, Methylaminoessigsäure, ε-Aminocapronsäure, Benzylamin, 2-, 3- oder 4-Chlorbenzylamin, 4-Methylbenzylamin, N-Methylbenzylamin, 2-, 3- oder 4-Sulfonbenzylamin, 2-Phenyläthylamin, 1-Phenyläthylamin, 1-Phenyl-2-propylamin, Cyclohexylamin, N-Methylcyclohexylamin, N-(2-Hydroxyäthyl)-cyclohexylamin, 2-, 3- oder 4-Methylcyclohexylamin, 3,3,5-Trimethylcyclohexylamin, Morpholin, Piperidin, Pyrrolidin, 1,2,3,4-Tetrahydroisochinolin.

Die Umsetzung der Difluortriazinylamino-hydroxy-naphthalinsulfonsäuren (5) mit den Aminen (6) erfolgt vorzugsweise sogleich im Anschluss an deren Herstellung durch Zugabe der 0–5° kalten wässrigen Lösung oder Suspension zu einer wässrigen Lösung des Amins (6) bei pH 8,5–12,5 bei 0–5°. Weiterhin ist es vorteilhaft, im Anschluss direkt das erhaltene Reaktionsgemisch bei 0–5° mit einer geeigneten Diazonium-Verbindung zu versetzen und bei dieser Temperatur und einem pH-Wert von 4–9 die Kupplung zu den Azofarbstoffen (7) herbeizuführen. Bevorzugt wird die Kupplung bei einem pH-Wert von 5–7,5 durchgeführt, der durch Zugabe säurebindender Mittel, wie verdünnte Natronlauge, Sodalösung oder Natriumphosphatlösung, eingehalten wird.

Die Verbindungen der Formel (8),

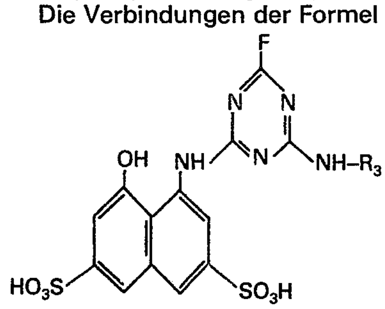

(8)

worin $R_3$ gegebenenfalls substituiertes $C_2$–$C_6$-Alkyl, $C_3$–$C_6$-Alkenyl oder Cycloalkyl oder substituiertes Aralkyl sind wertvolle Zwischenprodukte, die sich besonders zur H Azoreaktivfarbstoffe der Formel (9),

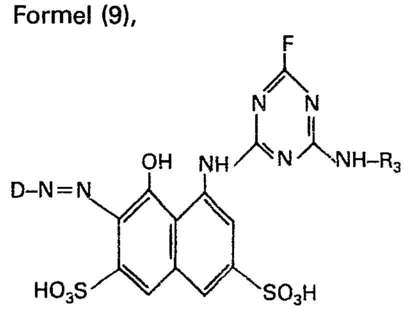

(9)

in der $R_3$ die oben angegebene Bedeutung hat und

D = Rest einer Diazokomponente ist, in der Weise eignen, dass man die reaktiven Zwischenprodukte (8) anschliessend mit diazotierten Aminen der allgemeinen Formel (10)

D–$NH_2$ (10)

kuppelt.

Die Verbindungen der Formel (11),

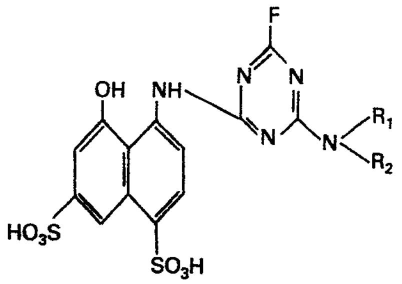

(11)

worin $R_1$ und $R_2$ die oben angegebene Bedeutung haben, sind ebenfalls wertvolle neue Zwischenprodukte, die sich besonders zur Herstellung der neuen Azoreaktivfarbstoffe der Formel (12),

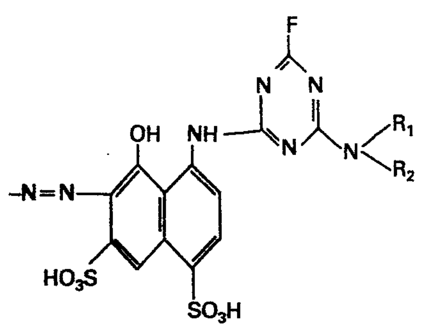

(12)

in der $R_1$ und $R_2$ die weiter oben angegebene Bedeutung haben, und D = Rest einer Diazokomponente ist, in der Weise eignen, dass man die reaktiven Zwischenprodukte (11) anschliessend mit diazotierten Aminen der allgemeinen Formel (10)

D–$NH_2$ (10)

kuppelt.

Geeignete Substituenten für die Alkylreste $R_1$, $R_2$, $R_3$ sind beispielsweise Hydroxy, Alkoxy, insbesondere $C_1$–$C_4$-Alkoxy, Sulfate, Acyloxy, insbesondere $C_1$–$C_4$-Alkylcarbonyloxy, Halogen, Cyan, Carboxy, Sulfo, Carbonsäureester, Carbonamid, gegebenenfalls substituiertes Amino.

Geeignete substituierte Aralkylreste sind insbesondere durch Halogen-, Alkyl- oder Sulfonsäuregruppen substituierte Benzylreste.

Als Amine (10) kommen insbesondere solche der Benzol- und Naphthalinreihe sowie der Benzol-azo-benzol-, Benzol-azo-naphthalin und Naphthalin-azo-naphthalinreihe in Frage, die gegebenenfalls metallhaltig sein können und insbesondere Cu, Cr, Co in komplexgebundener Form enthalten können. Bevorzugte Amine sind solche mit wasserlöslich-machenden Gruppen, insbesondere 1–3 Sulfogruppen.

Als diazotierbare Amine der Formel (10) seien beispielsweise genannt:

2-Aminobenzolsulfonsäure, 3-Aminobenzolsulfonsäure, 4-Aminobenzolsulfonsäure, 2-Aminobenzol-1,4-disulfonsäure, 4-Aminobenzol-1,3-disulfonsäure, 6-Amino-5-methylbenzol-1,3-disulfonsäure, 2-Amino-5-methylbenzolsulfonsäure, 2-Amino-5-methoxybenzolsulfonsäure, 2-Amino-4-chlorbenzolsulfonsäure, 2-Amino-5-chlorbenzolsulfonsäure, 2-Amino-5-acetylaminobenzolsulfonsäure, 2-Amino-4-acetylaminobenzolsulfonsäure, 3-Amino-4-methoxybenzolsulfonsäure, 3-Amino-4-methylbenzolsulfonsäure, 2-Amino-3,5-dimethylbenzolsulfonsäure, 4-Amino-5-methylbenzol-1,2-disulfonsäure, 2-Amino-4-sulfobenzoesäure, 2-Amino-5-sulfobenzoesäure, 2-Aminobenzoesäure, 4-Aminobenzoesäure, 1-Amino-2-, -3- oder-4-methylbenzol, 1-Aminobenzol, 1-Amino-2-, -3- oder -4-methoxybenzol, 1-Amino-2-trifluormethylbenzol, 2-Aminonaphthalin-1-sulfonsäure, 2-Aminonaphthalin-1,5-disulfonsäure, 2-Amino-naphthalin-1,7-disulfonsäure, 3-Aminonaphthalin-1,5-disulfonsäure, 3-Aminonaphthalin-2,6-disulfonsäure, 2-Aminonaphthalin-3,6-disulfonsäure, 7-Aminonaphthalin-1,3-disulfonsäure, 6-Aminonaphthalin-1,3-disulfonsäure, 4-Aminonaphthalin-1-sulfonsäure, 5-Aminonaphthalin-1-sulfonsäure, 6-Aminonaphthalin-1-sulfonsäure, 1-Aminonaphthalin-3,7-disulfonsäure, 4-Aminonaphthalin-1,3-disulfonsäure, 6-Aminonaphthalin -1,3,5-trisulfonsäure, 7-Aminonaphthalin-1,3,6-trisulfonsäure, 4-Aminonaphthalin-1,3,6-trisulfonsäure, 4-Aminophenylmethansulfonsäure, 1-Amino-3- oder -4-(sulfoacetyl)-aminobenzol, 1-Amino-2- oder -4-chlorbenzol.

Eine besonders vorteilhafte und wirtschaftliche Verfahrensvariante besteht darin, dass man alle Verfahrensschritte hintereinander ohne Isolierung der Zwischenstufen durchführt.

Die Farbstoffe sins äusserst wertvolle Produkte, die sich für verschiedenste Anwendungszwecke eignen. Sie finden bevorzugtes Interesse für das Färben von hydroxylgruppenhaltigen und stickstoffhaltigen Textilmaterialien, insbesondere für Textilmaterialien aus nativer und regenerierter Cellulose, in roten Tönen. Dank des reaktiven Fluorsubstituenten im Triazinring eignen sich die Produkte ausserordentlich gut als Reaktivfarbstoffe zum Färben von Cellulosematerialien nach den hierfür bekannt gewordenen Färbetechniken, insbesondere auch zum Färben dieser Materialien im Ausziehverfahren aus langer Flotte.

Die angegebenen Formeln sind die der freien Säuren. Zum Färben werden im allgemeinen deren Salze eingesetzt, insbesondere die Alkalisalze (bspw. Na-, K-, Li-Salze) oder auch die Ammoniumsalze. Die Farbstoffe werden im allgemeinen auch in Form der Salze isoliert.

Bei den Temperaturangaben handelt es sich um °C.

Beispiel 1

16.0 g 1-Amino-8-hydroxy-naphthalin-3,6-disulfonsäure werden in 250 ml Wasser bei pH 5,5 gelöst, auf 0–5° gekühlt und in 5 Minuten tropfenweise mit 4,4 ml Cyanurfluorid versetzt. Durch gleichzeitige Zugabe von 20%iger Natriumcarbonatlösung wird der pH-Wert bei 4,0–4,5 gehalten.

Man rührt noch einige Minuten bei dieser Temperatur und pH 4,0–4,5 nach.

Anschliessend tropft man die so erhaltene Lösung der Verbindung der Formel

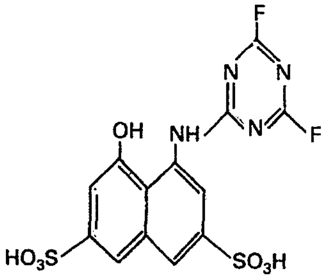

in eine 0–5° kalte Mischung von 25 ml 25%igen Ammoniak und 75 ml Wasser innerhalb von 15 Minuten ein. Der pH-Wert fällt während des Zutropfens von 12,0 auf 10,6. Nach 30 Minuten Rühren bei 0–5° wird der pH-Wert mit Salzsäure auf pH 7,0 gestellt. Die so erhaltene Lösung der Verbindung der Formel

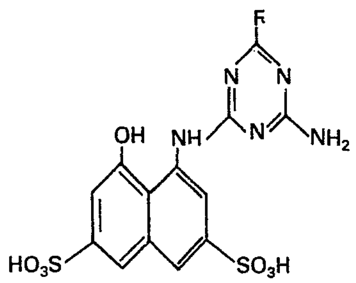

kann direkt nach der folgenden Vorschrift in einen brillanten roten Azoreaktivfarbstoff überführt werden.

Zu dem erhaltenen Reaktionsgemisch wird bei 0–5° eine Diazoniumsalz-Suspension gegeben, die durch Diazotierung von 8,7 g 2-Aminobenzolsulfonsäure in 100 ml Wasser und 15 ml 30%iger Salzsäure mit 25 ml 2n Natriumnitrit-Lösung bei 0–5° erhalten wurde, und der pH-Wert während des Eintropfens und während einer einstündigen Nachrührzeit (0–10°) mit 15%iger Natronlauge bei 6,5–7,5 gehalten. Nach Erwärmung auf Raumtemperatur wird die Fällung des auskristallisierten Reaktivfarbstoffs der Formel (als freie Säure)

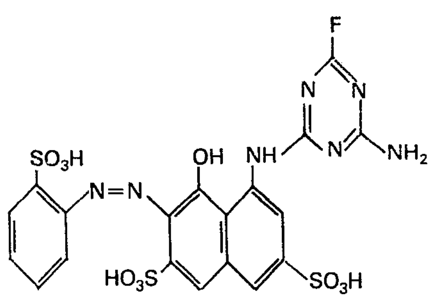

vervollständigt. Die Isolierung erfolgt durch Absaugen, Waschen mit wenig 15%iger Natriumchlorid-Lösung und Trocknung.

Beispiel 2

16,0 g 1-Amino-8-hydroxy-naphthalin-3,6-disulfonsäure werden in der im Beispiel 1, Absatz 1 angegebenen Weise mit Cyanurfluorid umgesetzt. Die Lösung der 1-(4′, 6′-Difluor-1′,3′,5′-triazin-2′-yl)-amino-8-hydroxy-naphthalin-3,6-disulfonsäure wird in eine 0–5° kalte Mischung von 7,75 g 30%iger Methylaminlösung und 100 ml Wasser, die durch Zugabe von etwas Salzsäure auf einen pH von 10,5 gestellt wurde, eingetropft. Der pH-Wert wird während des Eintropfens und während 15 Minuten Nachrühren (0–5°) durch Zutropfen 15%iger Natronlauge bei 10,5 gehalten. Anschliessend wird der pH-Wert mit Salzsäure auf pH 6,0 gestellt. Die so erhaltene Lösung der Verbindung der Formel

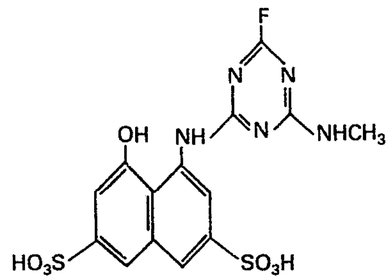

kann direkt nach der Vorschrift des Beipiels 1, Absatz 3 in den brillanten roten Azoreaktivfarbstoff der Formel (als freie Säure)

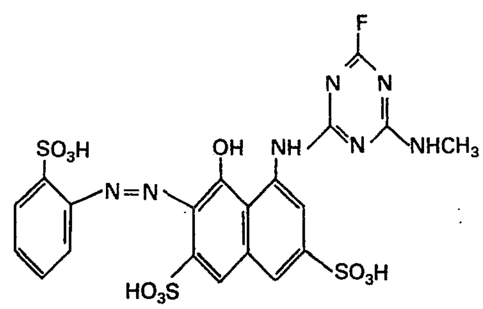

überführt werden.

Beispiel 3

Ersetzt man im vorangegangenen Beispiel die 7,75 g 30%iger Methylaminlösung durch 8,4 g 53,6%ige Dimethylaminlösung und verfährt sonst wie dort angegeben, so erhält man eine wässrige Lösung der Verbindung der Formel

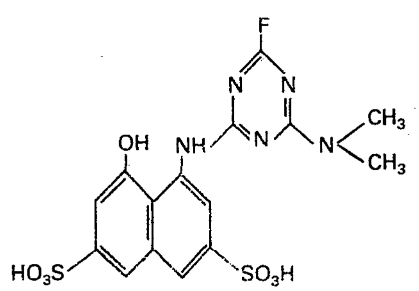

die nach der im Beispiel 1, Absatz 3 angegebene Vorschrift in den Azoreaktivfarbstoff der Formel (als freie Säure)

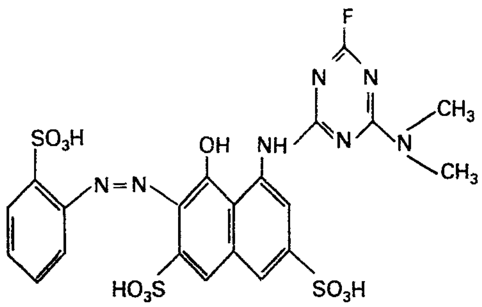

überführt werden kann.

Beispiel 4

16,0 g 1-Amino-8-hydroxy-naphthalin-3,6-disulfonsäure werden in der im Beispiel 1, Absatz 1 angegebenen Weise mit Cyanurfluorid umgesetzt. Die so erhaltene Lösung des Kondensationsproduktes wird in eine 0–5° kalte Mischung von 7,3 g n-Butylamin und 100 ml Wasser, die durch Zugabe von etwas Salzsäure auf pH 10,0 gebracht worden war, in 15 Minuten eingetropft. Hierbei wird der pH-Wert durch Zugabe 15%iger Natronlauge bei 10,0 gehalten. Nach 15 Minuten Nachrühren bei 0–5° wird der pH-Wert mit Salzsäure auf 6,0 gestellt. Die so erhaltene Lösung der Verbindung der Formel

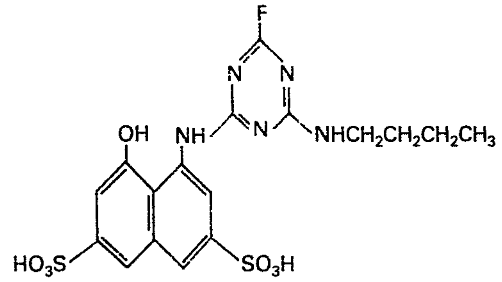

kann direkt nach der folgenden Vorschrift in einen blaustichig roten Azoreaktivfarbstoff überführt werden.

Zu dem Reaktionsgemisch wird bei 0–5° eine Diazoniumsalz-Suspension gegeben, die durch Diazotierung von 11,2 g 2-Amino-naphthalin-1-sulfonsäure in 150 ml Wasser und 15 ml 30%iger Salzsäure mit 25 ml 2n Natriumnitril-Lösung bei 0–5° erhalten wurde. Durch Zugabe von 15%iger Natronlauge wird der pH-Wert bei 6,5–7,0 gehalten. Nach 2 Stunden Rühren bei 0–5° wird auf Raumtemperatur erwärmt. Die Fällung des auskristallisierten Farbstoffs der Formel

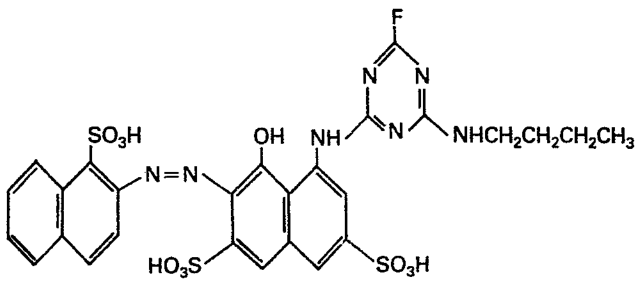

wird durch Zugabe von 50 g Natriumchlorid vervollständigt. Die Isolierung erfolgt durch Absaugen, Waschen mit wenig 15%iger Natriumchlorid-Lösung und Trocknen bei 60°.

50 g Baumwollstrang werden in 1 ltr Färbeflotte, die 1,5 g obigen Farbstoffes enthält, gefärbt, indem man innerhalb 30 Minuten auf 40° heizt, 50 g Natriumsulfat in mehreren Anteilen zugibt, anschliessend 20 g Soda zufügt und 60 Minuten bei dieser Temperatur behandelt.

Nach dem Spülen, kochendem Seifen und Trocknen erhält man eine sehr nassechte blausichtig rote Färbung mit guter Lichtechtheit.

Beispiel 5

16,0 g 1-Amino-8-hydroxy-naphthalin-3,6-disulfonsäure werden in der im Beispiel 1, Absatz 1 angegebenen Weise mit Cyanurfluorid umgesetzt. Die so erhaltene Lösung des Kondensationsproduktes wird in eine 0–5° kalte Mischung von 9,9 g Cyclohexylamin und 100 ml Wasser, die mit Salzsäure auf einen pH-Wert von 10,5 gestellt worden war, in 15 Minuten eingetropft. Hierbei wird der pH-Wert durch Zugabe 15%iger Natronlauge bei 10,5 gehalten. Nach 35 Minuten Nachrühren bei 0–5° und pH 10,5 wird mit HCl ein pH-Wert von 6,0 eingestellt. Die so erhaltene Lösung der Verbindung

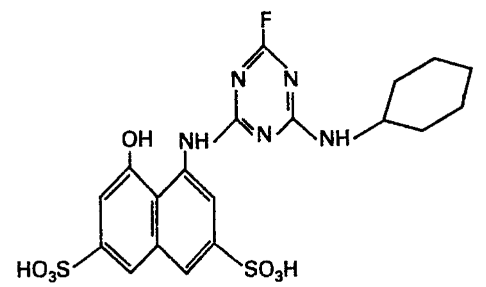

kann direkt in den folgenden Azoreaktivfarbstoff überführt werden,

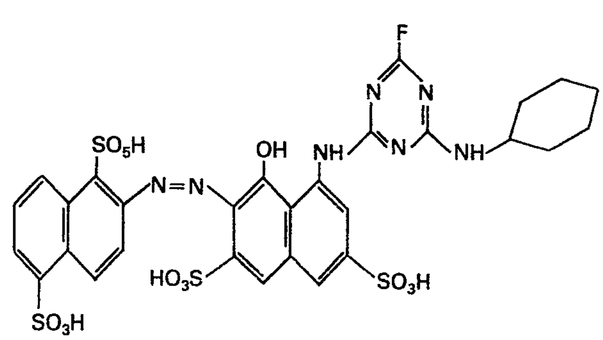

wenn nach der in Beispiel 1 angegebenen Methode unter Einsatz einer äquivalenten Menge 2-Amino-naphthalin-1,5-disulfonsäure an Stelle der 2-Amino-benzol-1-sulfonsäure verfahren wird.

Beispiel 6

16,0 g 1-Amino-8-hydroxy-naphthalin-4,6-disulfonsäure werden in 200 ml Wasser bei pH 6,0 gelöst und bei 0–5° innerhalb von 5 Minuten tropfenweise mit 4,4 ml Cyanurfluorid versetzt. Durch gleichzeitige Zugabe 20%iger Sodalösung wird der pH-Wert bei 4,0–4,5 gehalten. Nach 15 Minuten Nachrühren bei 0–5° und pH 4,0–4,5 wird die so erhaltene Lösung in eine Mischung aus 25 ml 25%igen Ammoniak, 100 ml Wasser und 100 g Eis, die durch Zugabe von etwas Salzsäure auf einen pH-Wert von 11,0 gebracht worden war, eingetropft. Hierbei fällt der pH-Wert auf 10,4 ab. Nach 15 Minuten Rühren bei 0–5° wird der pH-Wert mit Salzsäure auf 6,0 gebracht. Die so erhaltene Lösung der Verbindung der Formel

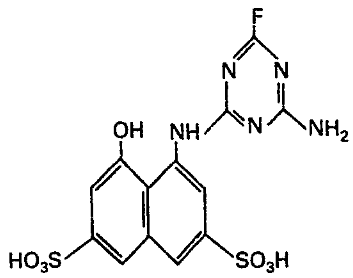

wird nach der in Beispiel 1, letztere Absatz angegebenen Vorschrift mit diazotierter 2-Amino-benzolsulfonsäure gekuppelt.

Der so erhaltene Farbstoff entspricht der Formel

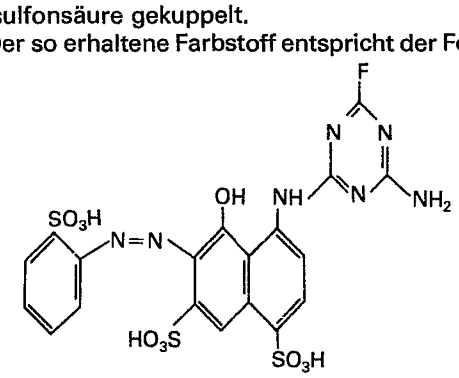

und färbt Baumwolle nach dem in Beispiel 4 angegebenen Verfahren in sehr nassechten roten Tönen.

Beispiel 7–23

Nach der in Beispiel 4 angegebenen Verfahrensweise lassen sich durch Kondensation von 1-Amino-8-hydroxy-naphthalin-3,6-disulfonsäure mit Cyanurfluorid und anschliessende Kondensation mit äquivalenten Mengen der Amine $R–NH_2$ weitere Zwischenprodukte der Formel I

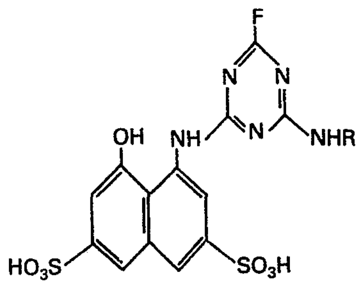

I

herstellen, die sich durch Kupplung mit den diazotierten Aminen $D–NH_2$ in die Farbstoffe der Formel II überführen lassen.

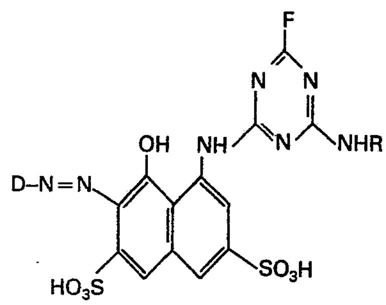

II

| Nr. | Amin $RNH_2$ | aromatisches Amin $DNH_2$ |
|---|---|---|
| 7 | Äthylamin | 2-Aminobenzol-1,4-disulfonsäure |
| 8 | Äthylamin | 4-Aminobenzol-1,3-disulfonsäure |
| 9 | Äthylamin | 2-Aminonaphthalin-1,5-disulfonsäure |
| 10 | Äthylamin | 2-Aminobenzol-1-sulfonsäure |
| 11 | n-Propylamin | 2-Aminobenzol-1,4-disulfonsäure |
| 12 | n-Propylamin | 2-Amino-4-acetylaminobenzolsulfonsäure |
| 13 | n-Propylamin | 3-Aminonaphthalin-1,5-disulfonsäure |
| 14 | Isopropylamin | 2-Aminobenzol-1-sulfonsäure |
| 15 | n-Butylamin | 4-Aminobenzol-1,3-disulfonsäure |
| 16 | n-Butylamin | 2-Aminonaphthalin-1,5-disulfonsäure |
| 17 | n-Butylamin | 2-Aminobenzol-1,4-disulfonsäure |
| 18 | n-Butylamin | 2-Amino-5-sulfobenzoesäure |
| 19 | n-Butylamin | 2-Amino-5-methoxybenzolsulfonsäure |
| 20 | sec. Butylamin | 2-Aminobenzol-1-sulfonsäure |
| 21 | Isobutylamin | 2-Aminobenzol-1-sulfonsäure |
| 22 | n-Hexylamin | 2-Aminobenzol-1-sulfonsäure |
| 23 | n-Hexylamin | 2-Aminonaphthalin-1,5-disulfonsäure |

Beispiel 24

16 g 1-Amino-8-hydroxy-naphthalin-3,6-disulfonsäure (= 0,05 Mol) werden in der im Beispiel 1, Absatz 1 angegebenen Weise mit Cyanurfluorid kondensiert. Die so erhaltene Lösung des Kondensationsproduktes wird in eine 0–5° kalte Mischung von 14,1 g Schwefelsäure-mono-(2-aminoäthyl)-ester («Aminolulfat»)(=0,1 Mol) und 100 ml Wasser, die durch Zugabe von etwas konzentrierter Natronlauge auf pH 10,5 gestellt wurde, eingetropft. Der pH-Wert wird während des Eintropfens und 15 Minuten Nachrühren (0–5°) durch Zugabe 15%iger Natronlauge bei 10,5 gehalten. Anschliessend wird der pH-Wert mit Salzsäure auf 6,0 gestellt. Die so erhaltene Lösung der Verbindung der Formel

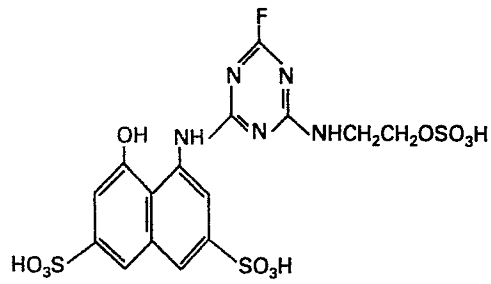

kann direkt zu dem folgenden Azoreaktivfarbstoff umgesetzt werden.

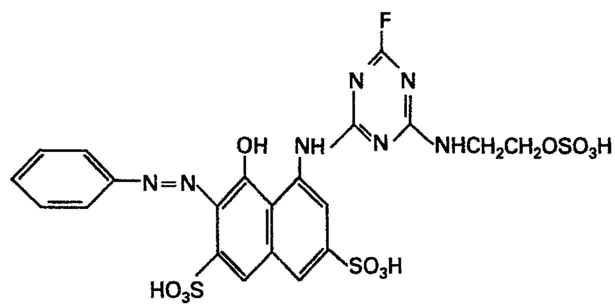

Hierzu wird zu dem Reaktionsgemisch bei 0–5° eine Diazoniumsalz-Lösung gegeben, die durch Diazotierung von 5,0 Anilin in 100 ml Wasser und 15 ml 30%iger Salzsäure und 25 ml 2n Natriumnitrit-Lösung bei 0–5° erhalten wurde, und der pH-Wert während des Eintropfens und einer zweistündigen Nachrührzeit (0–5°) durch laufende Zugabe 15%iger Natronlauge bei 6,0–7,0 gehalten. Nach 1 Stunde bei 20–30° wird die Fällung des Farbstoffes durch Zugabe von 100 g Natriumchlorid vervollständigt. Anschliessend wird abgesaugt, mit weniger 15%iger Natriumchlorid-Lösung gewaschen und bei 60° getrocknet. Der erhaltene Farbstoff färbt Baumwolle nach der im Beispiel 4 angegebenen Methode in blaustichig roten Tönen.

Beispiel 25

Verfährt man, wie in Beispiel 24 angegeben, und kuppelt anstelle mit diazotiertem Anilin mit der äquivalenten Menge diazotierter 2-Aminobenzolsulfonsäure, so erhält man einen roten Azoreaktivfarbstoff der Formel

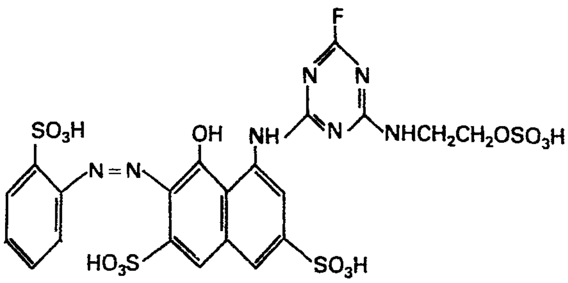

Beispiel 26–90

Nach der im Beispiel 4 oder 24 angegebenen Verfahrensweise lassen sich durch Kondensation von 16 g (= 0,05 Mol) 1-Amino-8-hydroxynaphthalin-3,6-disulfonsäure mit Cyanurfluorid und anschliessende Kondensation mit den Aminen $R-NH_2$ in der unten angegebenen Molmenge bei dem angegebenen pH-Wert weitere Zwischenprodukte der Formel I

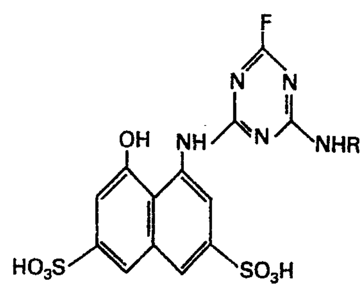

I

herstellen, die sich durch Kupplung mit den diazotierten Aminen $D-NH_2$ in die Farbstoffe der Formel II überführen lassen.

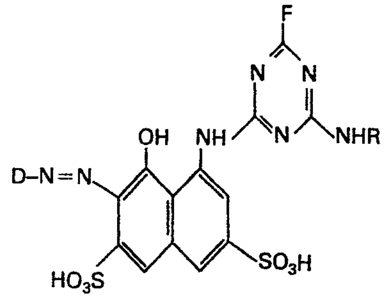

II,

| Nr. | Amin R–$NH_2$ | Mol $RNH_2$ | pH-Wert der Kondensation zu I | Arom. Amin D–$NH_2$ zu II |
|---|---|---|---|---|
| 26 | 2-Amino-äthanol | 0,1 | 10,0 | p-Toluidin |
| 27 | do. | 0,1 | 10,0 | o-Toluidin |
| 28 | do. | 0,075 | 10,5 | Anilin |
| 29 | do. | 0,075 | 10,5 | 2-Amino-benzolsulfonsäure |
| 30 | do. | 0,1 | 10,0 | 2-Aminonaphthalin-1-sulfonsäure |
| 31 | do. | 0,1 | 10,0 | 4-Aminobenzoesäure |
| 32 | do. | 0,1 | 10,0 | 2-Amino-4-acetylaminobenzolsulfonsäure |
| 33 | 2-Methoxy-äthylamin | 0,075 | 10,5 | 2-Amino-benzolsulfonsäure |
| 34 | do. | 0,075 | 10,5 | 4-Aminophenylmethansulfonsäure |
| 35 | 3-Amino-1-propanol | 0,1 | 10,0 | 2-Amino-5-methylbenzolsulfonsäure |
| 36 | do. | 0,1 | 10,0 | Anilin |
| 37 | do. | 0,1 | 10,0 | 2-Aminonaphthalin-1-sulfonsäure |
| 38 | do. | 0,065 | 11,0 | 2-Amino-5-methylbenzolsulfonsäure |
| 39 | 3-Methoxy-propylamin | 0,1 | 10,5 | 2-Amino-benzolsulfonsäure |
| 40 | Aminoessig-säure | 0,1 | 10,0 | Anilin |
| 41 | do. | 0,15 | 9,5 | 2-Aminobenzolsulfonsäure |
| 42 | Aminoessig- | 0,065 | 11,0 | 2-Aminonaphthalin-1-sulfonsäure |
| 43 | 3-Aminopro-pansäure | 0,1 | 10,5 | Anilin |
| 44 | do. | 0,1 | 10,5 | 2-Amino-5-methylbenzolsulfonsäure |
| 45 | 1-Amino-2-diäthylami-noäthan | 0,1 | 10,5 | Anilin |
| 46 | do. | 0,1 | 10,5 | 2-Aminobenzolsulfonsäure |
| 47 | 1-Amino-3-dimethyl-aminopropan | 0,1 | 10,0 | Anilin |
| 48 | do. | 0,075 | 10,5 | 2-Aminobenzolsulfonsäure |
| 49 | do. | 0,075 | 10,5 | 2-Aminonaphthalin-1-sulfonsäure |
| 50 | 6-Amino-hexansäure | 0,1 | 10,5 | 2-Aminobenzol-1,3-disulfonsäure |
| 51 | 2-Amino-äthansul-fonsäure | 0,1 | 10,0 | Anilin |
| 52 | do. | 0,1 | 10,0 | 2-Aminobenzolsulfonsäure |
| 53 | do. | 0,2 | 9,5 | p-Toluidin |
| 54 | do. | 0,1 | 10,0 | o-Chloranilin |
| 55 | do. | 0,1 | 10,0 | 2-Aminonaphthalin-1-sulfonsäure |
| 56 | 4-Aminobu-tansäure | 0,1 | 10,5 | Anilin |
| 57 | do. | 0,1 | 10,5 | 2-Aminobenzolsulfonsäure |
| 58 | 3-Methylcyclo-hexylamin | 0,075 | 10,5 | 2-Aminobenzol-1,4-disulfonsäure |
| 59 | do. | 0,1 | 10,0 | 2-Aminonaphthalin-1,5-disulfonsäure |
| 60 | Cyclohexylamin | 0,1 | 10,5 | 2-Aminobenzol-1,4-disulfonsäure |
| 61 | do. | 0,1 | 10,5 | 3-Aminonaphthalin-2,6-disulfonsäure |
| 62 | Aminomethylcyclo-hexan | 0,1 | 10,5 | 2-Aminobenzolsulfonsäure |
| 63 | 1-Amino-2-phenyl-äthan | 0,1 | 10,0 | 7-Aminonaphthalin-1,3,6-trisulfonsäure |
| 64 | Äthylamin | 0,075 | 10,5 | 1-Amino-4-(sulfoacetyl)-aminobenzol |
| 65 | do. | 0,1 | 10,0 | 2-Aminobenzoesäure |
| 66 | do. | 0,15 | 9,5 | 2-Amino-3,5-dimethylbenzolsulfonsäure |
| 67 | do. | 0,1 | 10,0 | 3-Aminobenzolsulfonsäure |

(Fortsetzung)

| Nr. | Amin R–$NH_2$ | Mol $RNH_2$ | pH-Wert der Kondensation zu I | Arom. Amin D–$NH_2$ zu II |
|---|---|---|---|---|
| 68 | Schwefelsäuremono(2-aminoäthyl)-ester | 0,1 | 10,5 | p-Toluidin |
| 69 | do. | 0,1 | 10,5 | 2-Amino-5-chlor-benzolsulfonsäure |
| 70 | do. | 0,06 | 11,0 | o-Toluidin |
| 71 | 2-Amino-äthanol | 0,1 | 10,0 | 2-Aminonaphthalin-1,7-disulfonsäure |
| 72 | do. | 0,1 | 10,0 | 2-Aminonaphthalin-3,6-disulfonsäure |
| 73 | do. | 0,1 | 10,0 | 4-Aminonaphthalin-1-sulfonsäure |
| 74 | 2-Aminoäthanol | 0,1 | 10,0 | 4-Aminonaphthalin-1,3-disulfonsäure |
| 75 | do. | 0,1 | 10,0 | 2-Amino-5-chlorbenzolsulfonsäure |
| 76 | Aminoessigsäure | 0,1 | 10,0 | 2-Amino-4-chlorbenzolsulfonsäure |
| 77 | do. | 0,1 | 10,0 | 5-Acetylamino-2-aminobenzolsulfonsäure |
| 78 | do. | 0,1 | 10,0 | 4-Acetylamino-2-aminobenzolsulfonsäure |
| 79 | 3-Aminopropanol | 0,08 | 10,5 | 2-Aminonaphthalin-1-sulfonsäure |
| 80 | do. | 0,1 | 10,0 | o-Chlor-anilin |
| 81 | do. | 0,1 | 10,0 | 2-Amino-4-acetylamino-benzolsulfonsäure |
| 82 | n-Propylamin | 0,075 | 11,0 | 3-Aminobenzolsulfonsäure |
| 83 | do. | 0,3 | 9,5 | 4-Aminobenzolsulfonsäure |
| 84 | do. | 0,2 | 10,0 | 6-Amino-5-methylbenzol-1,3-disulfonsäure |
| 85 | do. | 0,2 | 10,5 | 2-Amino-4-acetylaminobenzolsulfonsäure |
| 86 | do. | 0,2 | 9,5 | 2-Aminonaphthalin-1,7-disulfonsäure |
| 87 | do. | 0,4 | 9,0 | 4-Aminonaphthalin-1,3-disulfonsäure |
| 88 | 2-Acetylamino-äthylamin | 0,1 | 10,5 | 2-Aminonaphthalin-1-sulfonsäure |
| 89 | do. | 0,08 | 10,8 | 2-Aminobenzolsulfonsäure |
| 90 | 2-Propenyl-amin | 0,1 | 10,5 | 2-Aminobenzolsulfonsäure |

Beispiel 91–125

Nach der im Beispiel 6 angegebenen Verfahrensweise lassen sich durch Kondensation von 16 g (= 0,05 Mol) 1-Amino-8-hydroxynaphthalin-4,6-disulfonsäure mit Cyanurfluorid und anschliessende Kondensation mit den Aminen R–$NH_2$ in der unten angegebenen Molmenge bei dem angegebenen pH-Wert weitere Zwischenprodukte der Formel I

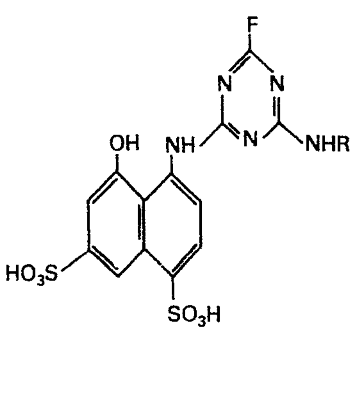

I

herstellen, die sich durch Kupplung mit den diazotierten Aminen D–$NH_2$ in die Farbstoffe der Formel II überführen lassen.

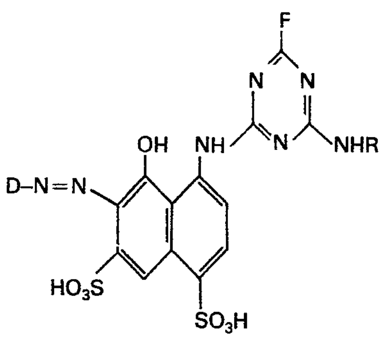

II

| Nr. | Amin $R-NH_2$ | Mol $RNH_2$ | pH-Wert der Kondensation zu I | Arom. Amin $D-NH_2$ zu II |
|---|---|---|---|---|
| 91 | Ammoniak | 0,3 | 11,0 | Anilin |
| 92 | do. | 0,2 | 11,5 | 2-Aminonaphthalin-1-sulfonsäure |
| 93 | Methylamin | 0,075 | 10,5 | Anilin |
| 94 | do. | 0,075 | 10,5 | 2-Aminobenzolsulfonsäure |
| 95 | do. | 0,1 | 10,0 | 2-Aminonaphthalin-1-sulfonsäure |
| 96 | Methylamin | 0,1 | 10,0 | 2-Amino-5-methylbenzolsulfonsäure |
| 97 | Dimethylamin | 0,1 | 10,5 | 2-Aminobenzolsulfonsäure |
| 98 | do. | 0,1 | 10,5 | 2-Aminonaphthalin-1-sulfonsäure |
| 99 | Diäthylamin | 0,15 | 9,5 | 2-Aminobenzol-1,3-disulfonsäure |
| 100 | 2-Aminoäthanol | 0,1 | 10,0 | o-Toluidin |
| 101 | 2-Aminoäthanol | 0,075 | 10,5 | Anilin |
| 102 | do. | 0,075 | 10,5 | 2-Amino-benzolsulfonsäure |
| 103 | do. | 0,1 | 10,0 | 2-Aminonaphthalin-1-sulfonsäure |
| 104 | 2-Methylamino-äthanol | 0,1 | 10,0 | Anilin |
| 105 | do. | 0,1 | 10,0 | p-Toluidin |
| 106 | Bis-(2-hydroxy-äthyl)-amin | 0,1 | 10,0 | Anilin |
| 107 | do. | 0,1 | 10,0 | 2-Aminonaphthalin-1-sulfonsäure |
| 108 | Cyclohexylamin | 0,1 | 10,5 | 7-Aminonaphthalin-1,3,6-trisulfonsäure |
| 109 | Benzylamin | 0,1 | 10,0 | 2-Aminonaphthalin-3,6-disulfonsäure |
| 110 | do. | 0,1 | 10,0 | 2-Aminonaphthalin-1,5-disulfonsäure |
| 111 | Schwefelsäure-mono(2-amino-äthyl)-ester | 0,1 | 10,5 | p-Toluidin |
| 112 | 3-Amino-1-propanol | 0,1 | 10,0 | 2-Amino-5-methylbenzolsulfonsäure |
| 113 | 3-Amino-1-propanol | 0,1 | 10,0 | Anilin |
| 114 | Methoxypropylamin | 0,1 | 10,5 | 2-Aminobenzolsulfonsäure |
| 115 | Methylamino-essigsäure | 0,1 | 10,0 | Anilin |
| 116 | Aminoessigsäure | 0,1 | 10,0 | Anilin |
| 117 | do. | 0,1 | 10,0 | 2-Aminobenzolsulfonsäure |
| 118 | 2-Aminoäthan-sulfonsäure | 0,1 | 10,0 | Anilin |
| 119 | do. | 0,1 | 10,0 | 2-Aminobenzolsulfonsäure |
| 120 | do. | 0,1 | 10,0 | 2-Aminonaphthalin-1-sulfonsäure |
| 121 | n-Butylamin | 0,075 | 10,5 | 2-Aminobenzolsulfonsäure |
| 122 | do. | 0,075 | 10,5 | 4-Aminobenzoesäure |
| 123 | do. | 0,1 | 10,0 | 2-Amino-5-methylbenzolsulfonsäure |
| 124 | Morpholin | 0,15 | 9,5 | 2-Aminobenzolsulfonsäure |
| 125 | 3-Methylamino-propannitril | 0,15 | 10,0 | 2-Aminobenzolsulfonsäure |
| 126 | 2-Methylamino-äthansulfon-säure | 0,1 | 10,0 | 2-Amino-5-methylbenzolsulfonsäure |
| 127 | Methyläthylamin | 0,15 | 9,5 | 3-Aminobenzolsulfonsäuredimethylamid |
| 128 | Piperidin | 0,1 | 10,0 | 2-Amino-4-sulfobenzoesäure |
| 129 | Hydroxyäthyl-cyclohexylamin | 0,1 | 10,0 | 2-Aminobenzolsulfonsäure |

**Patentansprüche**

1. Verfahren zur Herstellung von Verbindungen der Formel

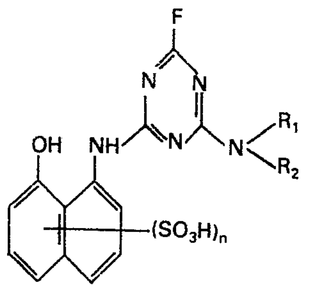

worin

$R_1$ = H, gegebenenfalls substituiertes Alkyl, Cycloalkyl oder Aralkyl,

$R_2$ = H, gegebenenfalls substituiertes Alkyl

n = 1, 2 wobei die Alkylreste $R_1$ und $R_2$ auch zu einem heterocyclischen Ring geschlossen sein können, dadurch gekennzeichnet, dass man eine Verbindung der Formel

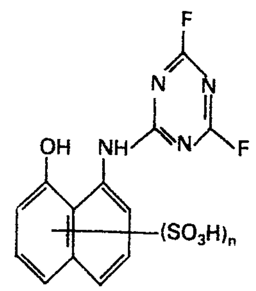

– vorzugsweise in wässriger Lösung oder Suspension – zu einer wässrigen Lösung oder Suspension eines Amins der Formel

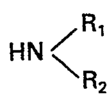

gibt und hierbei einen pH-Wert zwischen 8,5 und 12,5 einhält.

2. Verfahren nach Anspruch 1, dadurch gekennzeichnet, dass man 1–4 Mol Amin oder im Fall, dass $R_1$ und $R_2$ Wasserstoff sind, 1–10 Mol Amin verwendet.

3. Verfahren zur Herstellung von Verbindungen der Formel

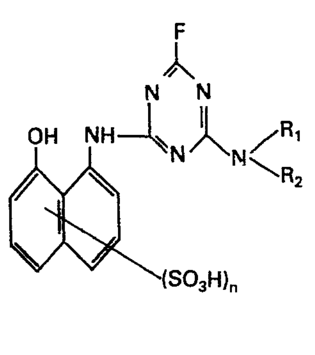

worin $R_1$, $R_2$ und n die in Anspruch 1 angegebene Bedeutung haben, dadurch gekennzeichnet, dass man zunächst 2,4,6-Trifluor-s-triazin in wässrigem Medium, vorzugsweise bei pH-Werten von 3,5–4,5 und Temperaturen von 0 bis 10 °C mit Aminen der Formel

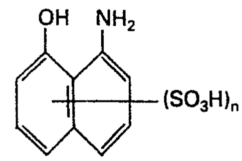

umsetzt und die dabei erhaltenen Reaktionslösungen unmittelbar anschliessend zu einer wässrigen Lösung
oder Suspension eines Amins der Formel

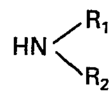

gibt und hierbei einen pH-Wert von 8,5–12,5 einhält.

4. Verbindungen der Formel

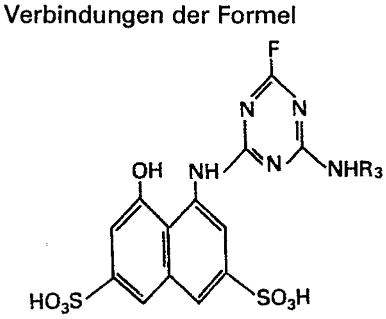

worin

$R_3$ gegebenenfalls substituiertes $C_2$–$C_6$-Alkyl, $C_3$–$C_6$-Alkenyl oder Cycloalkyl oder substituiertes Aralkyl
ist.

5. Verbindungen der Formel

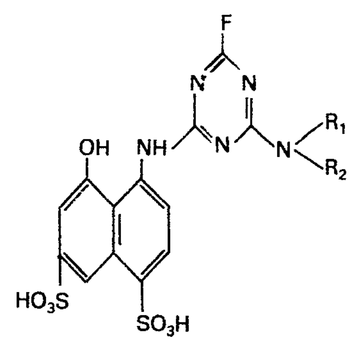

worin $R_1$ und $R_2$ die im Anspruch 1 angegebene Bedeutung haben.

6. Verfahren zur Herstellung von Farbstoffen der Formel

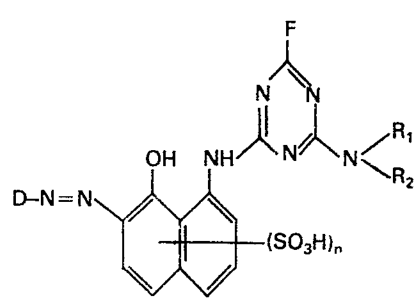

worin

$R_1$, $R_2$ und n die in Anspruch 1 angegebene Bedeutung haben und D = Rest einer Diazokomponente, dadurch gekennzeichnet, dass man die gemäss Anspruch 3 hergestellte Kupplungskomponente mit diazotierten Aminen der Formel $D\text{–}NH_2$ kuppelt, wobei D die oben angegebene Bedeutung besitzt.

7. Verfahren zum Färben und Bedrucken von hydroxylgruppenhaltigen und stickstoffhaltigen Materialien, insbesondere Cellulosematerialien, dadurch gekennzeichnet, dass man Farbstoffe, hergestellt gemäss Anspruch 6, verwendet.

**REVENDICATIONS**

1. Procédé de production de composés de formule:

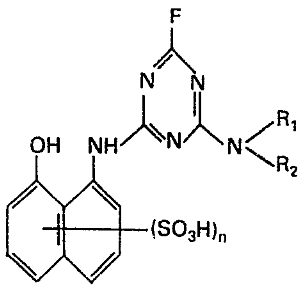

dans laquelle:

$R_1$ = H, un reste alkyle, cycloalkyle ou aralkyle éventuellement substitué,

$R_2$ = H, un reste alkyle éventuellement substitué,

n = 1, 2

les restes alkyle $R_1$ et $R_2$ pouvant aussi être cyclisés en un noyau hétérocyclique, caractérisé en ce qu'on ajoute un composé de formule:

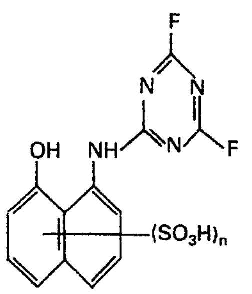

de préférence en solution ou en suspension aqueuse – à une solution ou suspension aqueuse d'une amine de formule:

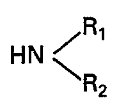

tout en maintenant une plage de pH de 8,5 à 12,5.

2. Procédé suivant la revendication 1, caractérisé en ce qu'on utilise 1–4 moles d'amine ou, lorsque $R_1$ et $R_2$ sont de l'hydrogène, 1–10 moles d'amine.

3. Procédé de production de composés de formule:

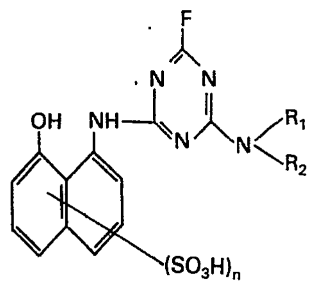

dans laquelle $R_1$, $R_2$ et n ont la définition indiquée dans la revendication 1, caractérisé en ce qu'on fait tout d'abord réagir de la 2,4,6-trifluoro-s-triazine en milieu aqueux, de préférence à des valeurs de pH de 3,5–4,5 et à des températures de 0 à 10 °C, avec des amines de formule:

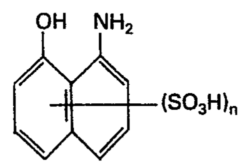

puis on ajoute immédiatement les solutions réactionnelles obtenues à une solution ou suspension aqueuse d'une amine de formule:

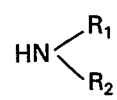

en maintenant une valeur de pH de 8,5–12,5.

4. Composés de formule:

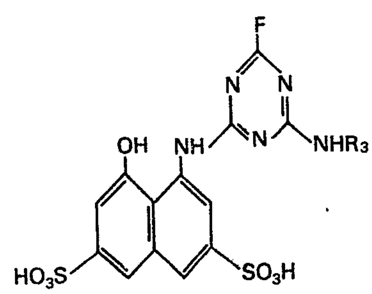

dans laquelle:

$R_3$ est un reste alkyle en $C_2$ à $C_6$ éventuellement substitué, alcényle en $C_3$ à $C_6$ ou cycloalkyle ou un reste aralkyle substitué.

5. Composés de formule:

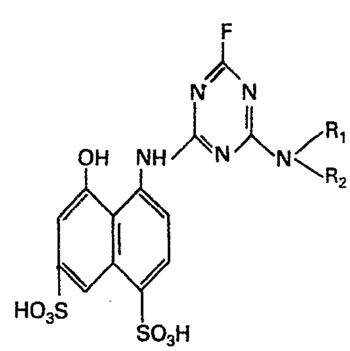

dans laquelle $R_1$ et $R_2$ ont la définition indiquée dans la revendication 1.

6. Procédé de production de colorants de formule:

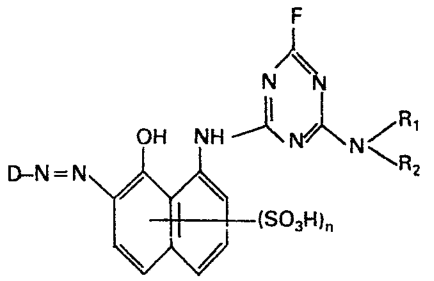

dans laquelle:

$R_1$, $R_2$ et n ont la définition indiquée dans la revendication 1 et D est le reste d'un composant diazotable, caractérisé en ce qu'on copule les copulants préparés conformément à la revendication 3 avec des amines diazotées de formule $D-NH_2$, D ayant la définition indiquée ci-dessus.

7. Procédé de teinture et d'impression de matières portant des groupes hydroxyle et contenant de l'azote, notamment des matières cellulosiques, caractérisé en ce qu'on utilise des colorants préparés conformément à la revendication 6.

**Claims**

1. Process for the preparation of compounds of the formula

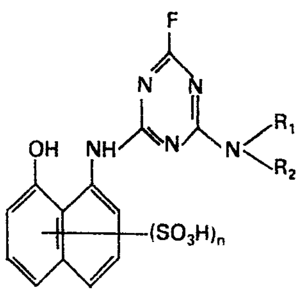

wherein

$R_1$ = H, optionally substituted alkyl, cycloalkyl or aralkyl,

$R_2$ = H or optionally substituted alkyl and

n = 1 or 2, and wherein the alkyl radicals $R_1$ and $R_2$ can also be closed to form a hererocyclic ring, characterised in that a compound of the formula

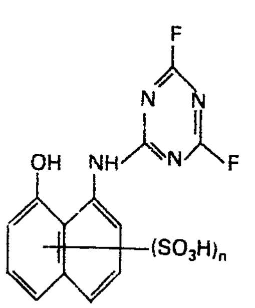

preferably in aqueous solution or suspension, is added to an aqueous solution or suspension of an amine of the formula

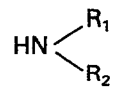

and the pH is kept in the range between 8.5 and 12.5 during this addition.

2. Process according to Claim 1, characterised in that 1–4 mols of amine or, in the case where $R_1$ and $R_2$ are hydrogen, 1–10 mols of amine are used.

3. Process for the preparation of compounds of the formula

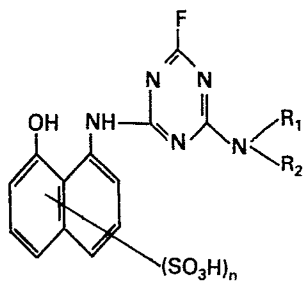

wherein

$R_1$, $R_2$ and n have the meaning indicated in Claim 1

characterised in that 2,4,6-trifluoro-s-triazine is first reacted with amines of the formula

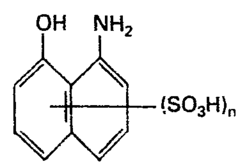

in an aqueous medium preferably at pH values of 3.5–4.5 and at temperatures of 0 to 10 °C, and the reaction solutions thereby obtained are then immediately added to an aqueous solution or suspension of an amine of the formula

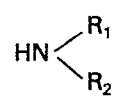

during which a pH value of 8.5–12.5 is maintained.

4. Compounds of the formula

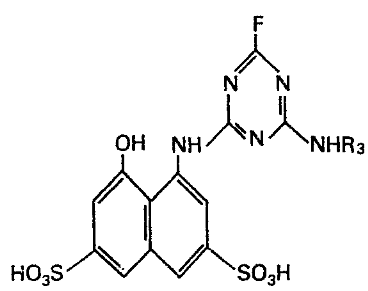

wherein

$R_3$ is optionally substituted $C_2$–$C_6$-alkyl, $C_3$–$C_6$-

alkenyl, cycloalkyl or substituted aralkyl.

5. Compounds of the formula

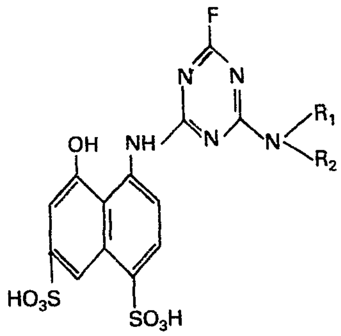

wherein

$R_1$ and $R_2$ have the meaning indicated in Claim 1.

6. Process for the preparation of dyestuffs of the formula

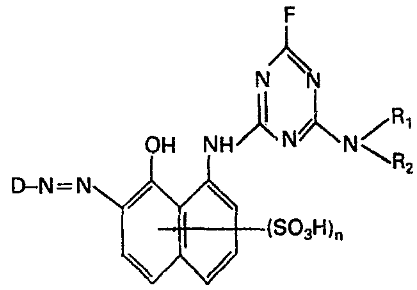

wherein

$R_1$, $R_2$ and n have the meaning indicated in Claim 1 and D = the radical of a diazo component, characterised in that the coupling component prepared according to Claim 3 is coupled with diazotised amines of the formula

$$D\text{–}NH_2$$

wherein

D has the meaning indicated above.

7. Process for dyeing and printing materials containing hydroxyl groups or nitrogen, in particular cellulose materials, characterised in that dyestuffs prepared according to Claim 6 are used.